# EUROPEAN PATENT APPLICATION

(11) **EP 4 104 840 A1**
(43) Date of publication of application: **21.12.2022**
(21) Application number: 21753522.8
(22) Date of filing: 09.02.2021
(51) Int. Cl.: A61K 35/14, A61P 35/00, C07K 16/28, C07K 14/705

(54) **OPTIMIZATION OF CHIMERIC ANTIGEN RECEPTOR**

(30) Priority: 13.02.2020 CN 202010090537
(71) Applicant: Beijing Immunochina Pharmaceuticals Co., Ltd., Beijing 100195 (CN)
(72) Inventor: LU, Xinan, Beijing 100195 (CN); HE, Ting, Beijing 100195 (CN); QI, Feifei, Beijing 100195 (CN); DING, Yanping, Beijing 100195 (CN); XIONG, Fuyin, Beijing 100195 (CN); LIANG, Mengmeng, Beijing 100195 (CN)
(74) Representative: karo IP
(86) International application number: PCT/CN2021/076231
(87) International publication number: WO 2021/160120

(57) **Abstract**

The present invention relates to the optimization of a chimeric antigen receptor, in particular, the modification of an intracellular co-stimulatory domain.

## Description

### Field of the Invention

The present invention relates to an optimization of a chimeric antigen receptor (CAR), in particular, a modification of an intracellular co-stimulatory domain.

### Background of the Invention

CAR-T technology combines the specificity of antibodies and the killing effect of T cells, thus forming an effective way of adoptive immunization (Benmebarek et al., Int. J. Mol. Sci. 20: 1283, 2019). The original CAR usually consists of an extracellular antigen-binding domain, a hinge region, a transmembrane region, and an intracellular signaling domain (Gross et al., Proc. Natl. Acad. Sci. USA 86: 10024-10028, 1989 ; Eshhar et al., Proc. Natl. Acad. Sci. USA 90: 720-724, 1993). The second-generation CAR adds an intracellular co-stimulatory domain, usually located between the transmembrane region and the intracellular signaling domain (Imai et al., Leukemia 18: 676-684, 2004 ; Zhao et al., Cancer Cell 28: 415-428, 2015). The third-generation CAR adds two co-stimulatory domains (Zhong et al., Mol. Ther. 18: 413-420, 2010). Although CAR-T technology has achieved great success in the treatment of hematological tumors, there are still some patients who do not respond or relapse after responding, and is still insufficient in the durability of efficacy and toxic side effects. In addition, CAR-T faces even more multiple obstacles such as limited efficacy and off-target effects in the treatment of solid tumors. Two important issues for the efficacy and safety of CAR-T are: 1) CAR-T amplification and persistence in vivo are insufficient; 2) CAR-T can cause toxic side effects such as cytokine storm, neurotoxicity, and off-target effects.

The reason for the above phenomenon is that the elements used in the current CAR molecules are basically wild-type, resulting in that the antigen recognition domain may not reach the optimal binding state for the formation of immune synapses when it binds to the target molecule, or that the signal transmission to the cell after the formation of the immune synapse is too strong or too weak, which thereby affects the survival, amplification of CAR-T cells in vivo, and the persistence of tumor cell killing performance and the regulation of the function of the entire immune system, which is ultimately reflected in the difference in clinical efficacy and safety.

By optimizing the CAR molecule, especially the intracellular co-stimulatory domain, the present invention improves the amplification efficiency and duration of the CAR-T cell in vivo, improves the tumor-killing efficiency, and reduces the toxic side effects, and ultimately achieves the purpose of improving clinical efficacy, reducing disease recurrence and reducing side effects.

### Summary of the Invention

In one aspect, the invention relates to a method of modifying a co-stimulatory domain from a member of the tumor necrosis factor receptor superfamily. In one embodiment, the method comprises replacing one or more amino acid residues within one or more motifs in the co-stimulatory domain that interact with a downstream signaling molecule. Optionally, the method may further comprise replacing one or more amino acid residues at the N-terminus and/or C-terminus of one or more motifs in the co-stimulatory domain that interact with a downstream signaling molecule. In another embodiment, the method comprises adding or deleting one or more motifs in the co-stimulatory domain that interact with a downstream signaling molecule. Optionally, the method may further comprise adding or deleting one or more amino acid residues at the N-terminus and/or C-terminus of the motif in the co-stimulatory domain that interacts with a downstream signaling molecule. In yet another embodiment, the method comprises adding to the co-stimulatory domain one or more multimerization (e.g., trimerization)-related sequences from a downstream signaling molecule. The present invention also relates to a modified co-stimulatory domain obtained by said method.

In one aspect, the invention relates to a modified co-stimulatory domain from a member of the tumor necrosis factor receptor superfamily. In one embodiment, the modified co-stimulatory domain comprises a replacement of one or more amino acid residues within one or more motifs that interact with a downstream signaling molecule. Optionally, the modified co-stimulatory domain may also comprise a replacement of one or more amino acid residues at the N-terminus and/or C-terminus of one or more motifs that interact with a downstream signaling molecule. In another embodiment, the modified co-stimulatory domain adds or deletes one or more motifs that interact with a downstream signaling molecule. Optionally, the modified co-stimulatory domain may also add or delete one or more amino acid residues at the N-terminus and/or C-terminus of one or more motifs that interact with a downstream signaling molecule. In yet another embodiment, one or more multimerization(e.g., trimerization)-related sequences from a downstream signaling molecule are added to the modified co-stimulatory domain.

In one aspect, the invention relates to a method of optimizing a chimeric antigen receptor comprising as a step the above method of modifying a co-stimulatory domain from a member of the tumor necrosis factor receptor superfamily. The method of optimizing a chimeric antigen receptor of the present invention (specifically, the method of modifying co-stimulatory a domain from a member of the tumor necrosis factor receptor superfamily) can be used alone, or can be used in combination with other methods for optimizing a chimeric antigen receptor (such as a method of modifying a signaling domain). The present invention also relates to an optimized chimeric antigen receptor obtained by said method.

In one aspect, the invention relates to an optimized chimeric antigen receptor comprising, as a component, the above modified co-stimulatory domain from a member of the tumor necrosis factor receptor superfamily. The optimized chimeric antigen receptor of the present invention may comprise not only the modified co-stimulatory domain from the member of the tumor necrosis factor receptor superfamily, but also the modified other components, such as the modified signaling domain.

In one aspect, the present invention relates to a chimeric antigen receptor comprising the modified co-stimulatory domain of the present invention. In one aspect, the present invention relates to an animal cell comprising the chimeric antigen receptor of the present invention. In one embodiment, the animal is a mammal (eg, canine, feline, porcine, bovine, ovine, goat, and equine). In one embodiment, the animal is a rodent (eg, mouse, rat, hamster, guinea pig, and rabbit) or a non-human primate (eg, monkey, ape, and chimpanzee). In one embodiment, the animal is a human. In one embodiment, the cell is a lymphocyte, such as a T cell, B cell or NK cell.

For example, the member of the tumor necrosis factor receptor superfamily is 4-1BB. For example, the downstream signaling molecule is TRAF2.

### Brief Description of Figures

Figure 1 shows CAR molecular elements and their effects on CAR-T function.
Figure 2 shows a schematic diagram of the vector pLenti-CAR.
Figure 3 shows a comparison of the proliferation rate of BCMA CAR-T cells with the modified 4-1BB co-stimulatory signaling domain.
Figure 4 shows a comparison of the ratios of CD4⁺ and CD8⁺ cells in BCMA CAR-T cells with the modified 4-1BB co-stimulatory signaling domain.
Figure 5 shows a comparison of the ratio of CCR7⁺CD62⁺ cells in BCMA CAR-T cells with the modified 4-1BB co-stimulatory signaling domain.
Figure 6 shows a comparison of the ratios of cells in a depleted state in BCMA CAR-T cells with the modified 4-1BB co-stimulatory signaling domain.
Figure 7 shows a comparison of the killing efficiency of BCMA CAR-T cells with the modified 4-1BB co-stimulatory signaling domain to tumor cells.

### Detailed Description

Unless otherwise indicated, the practice of the present invention will employ conventional techniques of molecular biology (including recombinant techniques), microbiology, cell biology, biochemistry and immunology, which are within the skill of the art. These techniques are fully explained in the literatures.

Unless otherwise defined, technical and scientific terms used herein have the same meaning as commonly understood by one of ordinary skill in the art to which this invention belongs.

### 1. Co-stimulatory domains

The intracellular co-stimulatory domains used in chimeric antigen receptors usually come from two families, the tumor necrosis factor receptor superfamily (TNFRSF) represented by 4-1BB and the CD28 family represented by CD28. Members of the tumor necrosis factor receptor superfamily include co-stimulatory molecules such as 4-1BB, ATAR, CD27, CD30, CD40, Ox40, LMP1, and LTβR. Their binding sites to a downstream signaling molecule (such as TRAF family members) are sequence conserved, including the primary consensus motif (P/S/A/T)X(Q/E)E and the secondary consensus motif PXQXXD, wherein X is any amino acid. For example, in the human sequence, 4-1BB (NP_001552.2) comprises TTQE and PEEE; ATRR comprises AVEE; CD27 comprises PIQE; CD30 comprises PEQE and SVEE; CD40 comprises PVQE; LMP1 comprises PQQATD; LTβR comprises PHQE; OX40 comprises PIQE; TANK comprises PIQCTD; TNF-R2 comprises SKEE. Various co-stimulatory molecules bind to TRAF2 of the TRAF family, but bind differently to other members of the TRAF family (e.g., TRAF1, TRAF3, TRAF5, TRAF6). See Hong et al., Molecular Cell 4: 321-330, 1999.

In the present invention, by modifying the motif(s) in the co-stimulatory domains from members of the tumor necrosis factor receptor superfamily that bind to a downstream signaling molecule, the proliferation efficiency of CAR-T cells in vivo is improved, the duration of CAR-T cells in vivo is prolonged, the killing efficacy of CAR-T cells on tumor cells is enhanced, cytokine release syndrome and neurotoxicity are reduced, and finally the purpose of improving clinical efficacy and reducing toxic and side effects is achieved.

In addition, in the co-stimulatory domains from members of the tumor necrosis factor receptor superfamily, sequence(s) that multimerize (eg, trimerize) the co-stimulatory domain is/are added to adjust the strength of downstream signaling. See Wu et al., Structural Studies of Human TRAF2, Cold Spring Harb Symp Quant Biol 64: 541-550, 1999.

In the present invention, 4-1BB may comprise, for example, the amino acid sequence listed in NP_001552.2, wherein the co-stimulatory domain corresponds to amino acid residues at positions of 214-255 (SEQ ID NO: 2). Alternatively, 4-1BB can be other naturally occurring homologous amino acid sequences, such as allelic products, isoforms, homologs, paralogs, and the like. Thus, the co-stimulatory domain from 4-1BB is the portion of the other naturally occurring homologous amino acid sequence corresponding to amino acid residues at positions of 214-255 of the amino acid sequence listed in NP_001552.2.

### 2. Method of modifying co-stimulatory domain

In one aspect, the invention relates to a method of modifying a co-stimulatory domain from a member of the tumor necrosis factor receptor superfamily, the method comprising replacing one or more amino acid residues within one or more motifs in the co-stimulatory domain that interact with a downstream signaling molecule. Optionally, the method may further comprise replacing one or more amino acid residues at the N-terminus and/or C-terminus of one or more motifs in the co-stimulatory domain that interact with a downstream signaling molecule.

In one aspect, the present invention relates to a method of modifying a co-stimulatory domain from a member of the tumor necrosis factor receptor superfamily, the method comprising adding or deleting one or more motifs in the co-stimulatory domain that interact with a downstream signaling molecule. Optionally, the method may further comprise adding or deleting one or more amino acid residues at the N-terminus and/or C-terminus of the motif in the co-stimulatory domain that interacts with a downstream signaling molecule.

In one aspect, the present invention relates to a method of modifying a co-stimulatory domain from a member of the tumor necrosis factor receptor superfamily, the method comprising adding to the co-stimulatory domain one or more multimerization (e.g., trimerization)-related sequences from a downstream signaling molecule.

In one embodiment, the member of the tumor necrosis factor receptor superfamily is selected from the group consisting of CD30, CD40, CD27, OX40, 4-1BB, ATAR, LTβR and LMP1. In one embodiment, the member of the tumor necrosis factor receptor superfamily is 4-1BB. In one embodiment, the downstream signaling molecule is selected from the group consisting of TRAF1, TRAF2, TRAF3, TRAF5 and TRAF6. In one embodiment, the downstream signaling molecule is TRAF2. In one embodiment, the member of the tumor necrosis factor receptor superfamily is 4-1BB and the downstream signaling molecule is TRAF2.

In one embodiment, the motif in the co-stimulatory domain from the member of the tumor necrosis factor receptor superfamily that interacts with a downstream signaling molecule is selected from the group consisting of (P/S/A/T)X(Q/E)E and PXQXXD, wherein X is any naturally occurring amino acid. In one embodiment, the motif in the co-stimulatory domain from a member of the member of the tumor necrosis factor receptor superfamily that interacts with a downstream signaling molecule is (P/S/A/T)X(Q/E)E, wherein X is any naturally occurring amino acid. In one embodiment, the replacement results in a non-naturally occurring motif. In one embodiment, a "non-naturally occurring motif" refers to a motif that is not naturally occurring in all tumor necrosis factor receptor superfamily members. In one embodiment, a "non-naturally occurring motif' refers to a non-naturally occurring motif in the modified tumor necrosis factor receptor superfamily member. In one embodiment, a "non-naturally occurring motif' refers to a motif that is not naturally occurring at the modified position in the modified tumor necrosis factor receptor superfamily member.

In one embodiment, the method comprises replacing one or more amino acid residues within two different motifs in the co-stimulatory domain that interact with downstream molecular signals, and resulting in the two different motifs to exchange positions. In one embodiment, the method further comprises replacing one or more amino acid residues at the N-terminus and/or C-terminus of two different motifs in the co-stimulatory domain, and resulting in a one or more amino acid residues at the N-terminus and/or C-terminus of two different motifs, together with the motif, to exchange positions.

In one embodiment, the TTQE motif (corresponding to positions 234-237 of the amino acid sequence listed in NP_001552.2) in the 4-1BB co-stimulatory domain (e.g. SEQ ID NO: 2, corresponding to positions 214-255 of the amino acid sequence listed in NP_001552.2) is replaced with a PTEE or PEEE motif. In one embodiment, the PEEE motif (corresponding to positions 246-249 in NP_001552.2) in the 4-1BB co-stimulatory domain (e.g. SEQ ID NO: 2, corresponding to positions 214-255 of the amino acid sequence listed in NP_001552.2) is replaced with a PEQE, TEQE or TTQE motif. In one embodiment, the PEEE motif in the 4-1BB co-stimulatory domain is replaced with a PEQE motif. In one embodiment, the TTQE motif in the 4-1BB co-stimulatory domain is replaced with a PTEE motif and the PEEE motif with a TEQE motif. In one embodiment, the TTQE motif in the 4-1BB co-stimulatory domain is replaced with a PEEE motif and the PEEE motif is replaced with a TTQE motif, i.e., the TTQE motif and the PEEE motif are exchanged in position. In one embodiment, the QTTQE sequence in the 4-1BB co-stimulatory domain is replaced by the FPEEE sequence and the FPEEE sequence is replaced by the QTTQE sequence, i.e., the TTQE motif along with the N-terminal Q residue and the PEEE motif with the N-terminal F residue are exchanged in position.

In one embodiment, TTQE and/or PEEE motifs are added to the 4-1BB co-stimulatory domain (e.g., SEQ ID NO: 2). In one embodiment, TTQE and/or PEEE motifs are added to the N- or C-terminus, particularly the C-terminus, of the 4-1BB co-stimulatory domain. In one embodiment, the VQTTQEEDGCS and/or RFPEEEEGGCE sequences are added to the 4-1BB co-stimulatory domain. In one embodiment, the VQTTQEEDGCS and/or RFPEEEEGGCE sequences are added to the N- or C-terminus, particularly the C-terminus, of the 4-1BB co-stimulatory domain.

In one embodiment, a DLAMADLEQKV trimerization sequence from TRAF2 is added to the 4-1BB co-stimulatory domain (e.g., SEQ ID NO: 2). In one embodiment, a DLAMADLEQKV trimerization sequence is added to the N- or C-terminus, particularly the C-terminus, of the 4-1BB co-stimulatory domain.

The present invention also relates to modified co-stimulatory domains obtained by said method.

### 3. Modified co-stimulatory domains

In one aspect, the invention relates to a modified co-stimulatory domain from a member of the tumor necrosis factor receptor superfamily, compared to a native sequence of the co-stimulatory domain, the modified co-stimulatory domain comprises a replacement of one or more amino acid residues within one or more motifs that interact with a downstream signaling molecule. Optionally, the modified co-stimulatory domain may also comprise a replacement of one or more amino acid residues at the N-terminus and/or C-terminus of one or more motifs that interact with a downstream signaling molecule.

In one aspect, the invention relates to a modified co-stimulatory domain from a member of the tumor necrosis factor receptor superfamily, compared to a native sequence of the co-stimulatory domain, the modified co-stimulatory domain adds or deletes one or more motifs that interact with a downstream signaling molecule. Optionally, the modified co-stimulatory domain may also add or delete one or more amino acid residues at the N-terminus and/or C-terminus of one or more motifs that interact with a downstream signaling molecule.

In one aspect, the invention relates to a modified co-stimulatory domain from a member of the tumor necrosis factor receptor superfamily, compared to a native sequence of the co-stimulatory domain, the modified co-stimulatory domain adds one or more multimerization (e.g., trimerization)-related sequences from a downstream signaling molecule.

In one embodiment, the member of the tumor necrosis factor receptor superfamily is selected from the group consisting of CD30, CD40, CD27, OX40, 4-1BB, ATAR, LTβR and LMP1. In one embodiment, the member of the tumor necrosis factor receptor superfamily is 4-1BB. In one embodiment, the downstream signaling molecule is selected from the group consisting of TRAF1, TRAF2, TRAF3, TRAF5 and TRAF6. In one embodiment, the downstream signaling molecule is TRAF2. In one embodiment, the member of the tumor necrosis factor receptor superfamily is 4-1BB and the downstream signaling molecule is TRAF2.

In one embodiment, the motif in the co-stimulatory domain from the member of the tumor necrosis factor receptor superfamily that interacts with a downstream signaling molecule is selected from the group consisting of (P/S/A/T)X(Q/E)E and PXQXXD, wherein X is any naturally occurring amino acid. In one embodiment, the motif in the co-stimulatory domain from the member of the tumor necrosis factor receptor superfamily that interacts with a downstream signaling molecule is (P/S/A/T)X(Q/E)E, wherein X is any naturally occurring amino acid. In one embodiment, the replacement results in a non-naturally occurring motif. In one embodiment, a "non-naturally occurring motif' refers to a motif that is not naturally occurring in all tumor necrosis factor receptor superfamily members. In one embodiment, a "non-naturally occurring motif' refers to a non-naturally occurring motif in the modified tumor necrosis factor receptor superfamily member. In one embodiment, a "non-naturally occurring motif' refers to a motif that is not naturally occurring at the modified position in the modified tumor necrosis factor receptor superfamily member.

In one embodiment, two different motifs exchange positions in the modified co-stimulatory domain. In one embodiment, two different motifs exchange positions in the modified co-stimulatory domain, and further, one or more amino acid residues at the N-terminus and/or C-terminus of the two different motifs, together with the motifs, exchange positions.

In one embodiment, compared to the native sequence 4-1BB co-stimulatory domain (e.g., SEQ ID NO: 2, corresponding to positions 214-255 of NP_001552.2), the modified 4-1BB co-stimulatory domain comprises PTEE or PEEE motif replaced from TTQE motif (corresponding to positions 234-237 of NP_001552.2). In one embodiment, compared to the native sequence 4-1BB co-stimulatory domain (e.g., SEQ ID NO: 2, corresponding to positions 214-255 of NP_001552.2), the modified 4-1BB co-stimulatory domain comprises PEQE, TEQE or TTQE motif replaced from PEEE motif (corresponding to positions 246-249 of NP_001552.2). In one embodiment, the modified 4-1BB co-stimulatory domain comprises PEQE motif replaced from PEEE motif. In one embodiment, the modified 4-1BB co-stimulatory domain comprises PTEE motif replaced from TTQE motif and TEQE motif replaced from PEEE motif. In one embodiment, the modified 4-1BB co-stimulatory domain comprises PEEE motif replaced from TTQE motif and TTQE motif replaced from PEEE motif. In one embodiment, the modified 4-1BB co-stimulatory domain comprises FPEEE motif replaced from QTTQE motif and QTTQE motif replaced from FPEEE motif. In one embodiment, the modified 4-1BB co-stimulatory domain comprises, consists essentially of, or consists of the amino acid sequence set forth in SEQ ID NO: 4, 6 or 8.

In one embodiment, compared to the native sequence 4-1BB co-stimulatory domain (e.g., SEQ ID NO: 2), the modified 4-1BB co-stimulatory domain comprises additional TTQE and/or PEEE motifs. In one embodiment, additional TTQE and/or PEEE motifs are added at the N- or C-terminus, especially the C-terminus, of the 4-1BB co-stimulatory domain. In one embodiment, the modified 4-1BB co-stimulatory domain comprises additional VQTTQEEDGCS and/or RFPEEEEGGCE sequences. In one embodiment, additional VQTTQEEDGCS and/or RFPEEEEGGCE sequences are added at the N- or C-terminus, especially the C-terminus, of the 4-1BB co-stimulatory domain. In one embodiment, the modified 4-1BB co-stimulatory domain comprises, consists essentially of, or consists of the amino acid sequence set forth in SEQ ID NO: 10 or 12.

In one embodiment, compared to the native sequence 4-1BB co-stimulatory domain (e.g., SEQ ID NO: 2), the modified 4-1BB co-stimulatory domain comprises the DLAMADLEQKV trimerization sequence from TRAF2. In one embodiment, the DLAMADLEQKV trimerization sequence is added at the N- or C-terminus, especially the C-terminus, of the 4-1BB co-stimulatory domain. In one embodiment, the modified 4-1BB co-stimulatory domain comprises, consists essentially of, or consists of the amino acid sequence set forth in SEQ ID NO: 14.

### 4. Methods for optimizing chimeric antigen receptors

In one aspect, the invention relates to a method of optimizing a chimeric antigen receptor comprising as a step the above method of modifying a co-stimulatory domain from a member of the tumor necrosis factor receptor superfamily, in particular 4-1BB.

The method of optimizing the chimeric antigen receptor of the present invention (specifically, the method of modifying the co-stimulatory domain from a member of the tumor necrosis factor receptor superfamily (especially 4-1BB)) can be used alone or in combination with other methods for optimizing chimeric antigen receptors (such as a method of modifying the signaling domain).

The present invention also relates to optimized chimeric antigen receptors obtained by said method.

### 5. Optimized chimeric antigen receptors

In one aspect, the present invention relates to an optimized chimeric antigen receptor comprising as a component the above modified co-stimulatory domain from a member of the tumor necrosis factor receptor superfamily, in particular 4-1BB.

The optimized chimeric antigen receptors of the present invention may comprise not only modified co-stimulatory domains from members of the tumor necrosis factor receptor superfamily, particularly 4-1BB, but also other modified component(s), such as a modified signaling domain.

In one embodiment, the present invention provides an optimized chimeric antigen receptor for BCMA comprising :
(1) an antigen binding domain for BCMA, particularly amino acids at positions of 23 to 268 of SEQ ID NO: 16 ;
(2) a hinge region, particularly amino acids at positions of 269 to 313 of SEQ ID NO: 16 ;
(3) a transmembrane region, particularly amino acids at positions of 314 to 337 of SEQ ID NO: 16 ;
(4) a co-stimulatory domain, particularly an amino acid sequence selected from the group consisting of SEQ ID NOs: 4, 6, 8, 10, 12 and 14 ; and
(5) a signaling domain, particularly amino acids at positions of 380 to 491 of SEQ ID NO:16.

Unless otherwise specified, the technical solutions described herein can be combined arbitrarily.

### Sequence Description

| SEQ ID NO | Description | Sequence |
|---|---|---|
| 1 | nucleotide sequence of 4-1BB co-stimulatory signaling domain of BCMA CAR | AAGCGCGGCCGCAAGAAGCTGCTGTACATCTTCAAGCAGCC CTTCATGCGCCCCGTGCAGACCACCCAGGAGGAGGACGGCT GCAGCTGCCGCTTCCCCGAGGAGGAGGAGGGCGGCTGCGA GCTG |
| 2 | amino acid sequence of 4-1BB co-stimulatory signaling domain of BCMA CAR | KRGRKKL LYIFKQPFMR PVQTTQEEDG CSCRFPEEEE GGCEL |
| 3 | nucleotide sequence of BCMA CAR-B5 co-stimulatory signaling domain | AAGCGCGGCCGCAAGAAGCTGCTGTACATCTTCAAGCAGCC CTTCATGCGCCCCGTGCAGACCACCCAGGAGGAGGACGGCT GCAGCTGCCGCTTCCCCGAGCAGGAGGAGGGCGGCTGCGA GCTG |
| 4 | amino acid sequence of BCMA CAR-B5 co-stimulatory signaling domain (E35Q) | KRGRKKL LYIFKQPFMR PVQTTQEEDG CSCRFPEQEE GGCEL |
| 5 | nucleotide sequence of BCMA CAR-B6 co-stimulatory signaling domain | AAGCGCGGCCGCAAGAAGCTGCTGTACATCTTCAAGCAGCC CTTCATGCGCCCCGTGCAGCCCACCGAGGAGGAGGACGGCT GCAGCTGCCGCTTCACCGAGCAGGAGGAGGGCGGCTGCGA GCTG |
| 6 | amino acid sequence of BCMA CAR-B6 co-stimulatory signaling domain (T21P, Q23E, P33T, E35Q) | KRGRKKL LYIFKQPFMR PVQPTEEEDG CSCRFTEQEE GGCEL |
| 7 | nucleotide sequence of BCMA CAR-B7 co-stimulatory signaling domain | AAGCGCGGCCGCAAGAAGCTGCTGTACATCTTCAAGCAGCC CTTCATGCGCCCCGTGTTCCCCGAGGAGGAGGAGGACGGCT GCAGCTGCCGCCAGACCACCCAGGAGGAGGGCGGCTGCGA GCTG |
| 8 | amino acid sequence of BCMA CAR-B7 co-stimulatory signaling domain (Q20F, T21P, T22E, Q23E, F32Q, P33T, E34T, E35Q) | KRGRKKL LYIFKQPFMR PVFPEEEEDG CSCRQTTQEE GGCEL |
| 9 | nucleotide sequence of BCMA CAR-B8 co-stimulatory signaling domain | AAGCGCGGCCGCAAGAAGCTGCTGTACATCTTCAAGCAGCC CTTCATGCGCCCCGTGCAGACCACCCAGGAGGAGGACGGCT GCAGCTGCCGCTTCCCCGAGGAGGAGGAGGGCGGCTGCGA GCTGGTGCAGACCACCCAGGAGGAGGACGGCTGCAGC |
| 10 | amino acid sequence of BCMA CAR-B8 co-stimulatory signaling domain (C-terminal part 4-1BB sequence) | KRGRKKL LYIFKQPFMR PVQTTQEEDG CSCRFPEEEE GGCEL RFPEEEE GGCE |
| 11 | nucleotide sequence of BCMA CAR-B9 co-stimulatory signaling domain | AAGCGCGGCCGCAAGAAGCTGCTGTACATCTTCAAGCAGCC CTTCATGCGCCCCGTGCAGACCACCCAGGAGGAGGACGGCT GCAGCTGCCGCTTCCCCGAGGAGGAGGAGGGCGGCTGCGA GCTGCGCTTCCCCGAGGAGGAGGAGGGCGGCTGCGAG |
| 12 | amino acid sequence of BCMA | KRGRKKL LYIFKQPFMR PVQTTQEEDG CSCRFPEEEE |
| | CAR-B9 co-stimulatory signaling domain (C-terminal part 4-1BB sequence) | GGCELVQTTQEEDGCS |
| 13 | nucleotide sequence of BCMA CAR-B11 co-stimulatory signaling domain | AAGCGCGGCCGCAAGAAGCTGCTGTACATCTTCAAGCAGCC CTTCATGCGCCCCGTGCAGACCACCCAGGAGGAGGACGGCT GCAGCTGCCGCTTCCCCGAGGAGGAGGAGGGCGGCTGCGA GCTGGACCTGGCGATGGCTGACTTGGAGCAGAAGGTC |
| 14 | amino acid sequence of BCMA CAR-B11 co-stimulatory signaling domain (C-terminal part TRAF2 sequence) | KRGRKKL LYIFKQPFMR PVQTTQEEDG CSCRFPEEEE GGCEL DLAMADLEQKV |
| 15 | nucleotide sequence of BCMA CAR molecule | ATGCTGCTGCTGGTGACCAGCCTGCTGCTGTGTGAGTTACCA CACCCAGCATTCCTCCTGATCCCAGACATTGTGCTCACTCAG TCACCTCCCAGCCTGGCCATGAGCCTGGGAAAAAGGCCAC CATCTCCTGTAGAGCCAGTGAGTCCGTCACAATCTTGGGGA GCCATCTTATTCACTGGTATCAGCAGAAGCCCGGGCAGCCTC CAACCCTTCTTATTCAGCTCGCGTCAAACGTCCAGACGGGTG TACCTGCCAGATTTTCTGGTAGCGGGTCCCGCACTGATTTA CACTGACCATAGATCCAGTGGAAGAAGACGATGTGGCCGTG TATTATTGTCTGCAGAGCAGAACGATTCCTCGCACATTTGGT GGGGGTACTAAGCTGGAGATTAAGGGAAGCACGTCCGGCT CAGGGAAGCCGGGCTCCGGCGAGGGAAGCACGAAGGGGC AAATTCAGCTGGTCCAGAGCGGACCTGAGCTGAAAAAACCC GGCGAGACTGTTAAGATCAGTTGTAAAGCATCTGGCTATAC CTTCACCGACTACAGCATAAATTGGGTGAAACGGGCCCCTG GAAAGGGCCTCAAATGGATGGGTTGGATCAATACCGAAACT AGGGAGCCTGCTTATGCATATGACTTCCGCGGGAGATTCGC CTTTTCACTCGAGACATCTGCCTCTACTGCTTACCTCCAAATA AACAACCTCAAGTATGAAGATACAGCCACTTACTTTTGCGCC CTCGACTATAGTTACGCCATGGACTACTGGGGACAGGGAAC CTCCGTTACCGTCAGTTCCACCACCACCCCAGCCCCCCGACC ACCAACACCCGCCCCCACCATCGCCAGCCAGCCCCTGAGCCT GCGCCCCGAGGCCTGCCGCCCCGCCGCCGGCGGCGCCGTGC ACACCCGCGGCCTGGACTTCGCCTGCGACATCTACATCTGG GCCCCCCTGGCCGGCACCTGCGGCGTGCTGCTGCTGAGCCT GGTGATCACCCTGTACTGCAAGCGCGGCCGCAAGAAGCTGC TGTACATCTTCAAGCAGCCCTTCATGCGCCCCGTGCAGACCA CCCAGGAGGAGGACGGCTGCAGCTGCCGCTTCCCCGAGGA GGAGGAGGGCGGCTGCGAGCTGCGCGTGAAGTTCAGCCGC AGCGCCGACGCCCCCGCCTACAAGCAGGGCCAGAACCAGCT GTACAACGAGCTGAACCTGGGCCGCCGCGAGGAGTACGAC GTGCTGGACAAGCGCCGCGGCCGCGACCCCGAGATGGGCG GCAAGCCCCGCCGCAAGAACCCCCAGGAGGGCCTGTACAAC GAGCTGCAGAAGGACAAGATGGCCGAGGCCTACAGCGAGA TCGGCATGAAGGGCGAGCGCCGCCGCGGCAAGGGCCACGA CGGCCTGTACCAGGGCCTGAGCACCGCCACCAAGGACACCT ACGACGCCCTGCACATGCAGGCCCTGCCCCCCCGCTAA |
| 16 | amino acid sequence of BCMA CAR molecule | MLLLVTSLLLCELPHPAFLLIPDIVLTQSPPSLAMSLGKRATISCR ASESVTILGSHLIHWYQQKPGQPPTLLIQLASNVQTGVPARFSG SGSRTDFTLTIDPVEEDDVAVYYCLQSRTIPRTFGGGTKLEIKGS TSGSGKPGSGEGSTKGQIQLVQSGPELKKPGETVKISCKASGYT FTDYSINWVKRAPGKGLKWMGWINTETREPAYAYDFRGRFAF SLETSASTAYLQINNLKYEDTATYFCALDYSYAMDYWGQGTSV TVSSTTTPAPRPPTPAPTIASQPLSLRPEACRPAAGGAVHTRGL DFACDIYIWAPLAGTCGVLLLSLVITLYCKRGRKKLLYIFKQPFMR PVQTTQEEDGCSCRFPEEEEGGCELRVKFSRSADAPAYKQGQN QLYNELNLGRREEYDVLDKRRGRDPEMGGKPRRKNPQEGLYN ELQKDKMAEAYSEIGMKGERRRGKGHDGLYQGLSTATKDTYD ALHMQALPPR |

### Examples

### Example 1 : Co-stimulatory signaling domain modification and optimization

This example takes, as an example, the modification and optimization of the 4-1BB co-stimulatory domain of the CAR (BCMA CAR) molecule targeting B cell maturation antigen (BCMA) constructed by the single-chain antibody in the Bluebird patent (US20180085444A1). Modification principle : without changing the nature and function of the co-stimulatory domain of 4-1BB, reducing the possibility of self-activation of CAR-T cells, increasing the efficiency of 4-1BB signaling into cells, promoting CAR-T cells to form more central memory T cells (CAR-Tcm), naive T cells (CAR-Tnaive), stem cell-like memory T cells (CAR-Tscm) subsets (collectively referred to as Tcns), extending the survival time of CAR-T cells in vivo, increasing the antitumor activity of CAR-T cells.

The nucleotide sequence of the initial BCMA CAR molecule is set forth in SEQ ID NO: 15, and the amino acid sequence is set forth in SEQ ID NO: 16.

For the unmodified 4-1BB co-stimulatory signaling domain, the nucleotide sequence of the 4-1BB co-stimulatory signaling domain of the BCMA CAR is set forth in SEQ ID NO: 1, and the amino acid sequence is set forth in SEQ ID NO: 2.

For the modified 4-1BB co-stimulatory signaling domain, the nucleotide sequence of the BCMA CAR-B5 co-stimulatory signaling domain is set forth in SEQ ID NO: 3, and the amino acid sequence is set forth in SEQ ID NO: 4; the nucleotide sequence of the BCMA CAR-B6 co-stimulatory signaling domain is set forth in SEQ ID NO: 5, and the amino acid sequence is set forth in SEQ ID NO: 6; the nucleotide sequence of the BCMA CAR-B7 co-stimulatory signaling domain is set forth in SEQ ID NO: 7, and the amino acid sequence is set forth in SEQ ID NO: 8; the nucleotide sequence of the BCMA CAR-B8 co-stimulatory signaling domain is set forth in SEQ ID NO: 9, and the amino acid sequence is set forth in SEQ ID NO: 10; the nucleotide sequence of the BCMA CAR-B9 co-stimulatory signaling domain is set forth in SEQ ID NO: 11, and the amino acid sequence is set forth in SEQ ID NO: 12; the nucleotide sequence of the BCMA CAR-B11 co-stimulatory signaling domain is set forth in SEQ ID NO: 13, and the amino acid sequence is set forth in SEQ ID NO: 14.

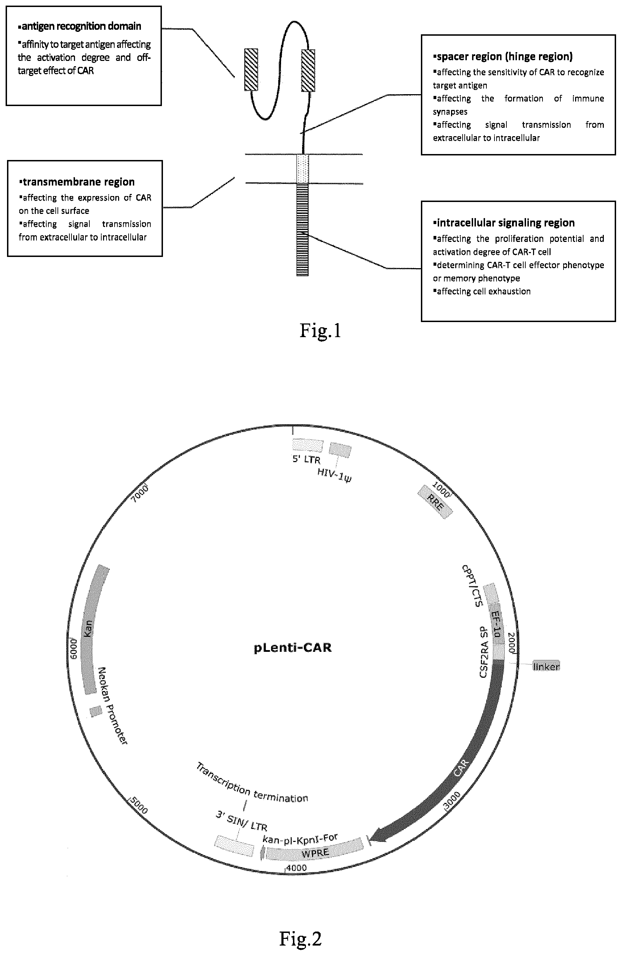

The nucleotide sequences of the above embodiments are synthesized by gene synthesis, and then bridged with other sequences to finally form a CAR coding molecule. The CAR encoding molecule was inserted into the lentiviral vector pLenti6.3/V5 (Thermo Fisher, Waltham, MA, USA) (Figure 2).

T cells were isolated from peripheral blood mononuclear cells of healthy volunteers (Miaotong (Shanghai) Biotechnology Co., Ltd., China). The isolated and purified T cells were seeded and cultured at 1.0 x 10⁶ cells/ml into X-VIVO 15 medium (Lonza, Switzerland), CD3/CD28 Dynabeads (Thermo Fisher) were added into the culture system according to the ratio of T cells to Dynabeads at 1:1, and after IL-2 (Shandong Jintai Bioengineering Co., Ltd., China) (500 IU/ml) was added and cultured for 48 hours, the CAR containing unmodified or modified 4-1BB was transduced into T cells by lentivirus. 24 hours after virus infection of cells, the cells were centrifuged to change the medium, and fresh X-VIVO 15 containing IL-2 (500 IU/ml) was added to continue the culture. After 4 days of cell culture, all cells in the culture system were collected, and the Dynabeads in the culture system were removed with a magnetic stand, T cells were centrifuged and counted, and after the CAR content of the cells in each group was detected by a flow cytometer (NovoCyte 2060R, ACEA Biosciences, San Diego, CA 92121, USA), NCI H929 was added for co-culture according to the ratio of CAR-T and NCI H929 cells (National Experimental Cell Resource Sharing Platform, China) at 5:1. The CAR-T cells were cultured until the 16th day, and the corresponding cell samples were collected for flow cytometry to analyze the CAR expression rate, the proportion of CD4⁺ cells, CD8⁺ cells, CCR7⁺CD62L⁺ cells in the CAR-T cells, and the CAR-T cell immune depletion molecule PD1, LAG3 expression, and tumor cell killing experiments were performed, basically as previously described (Sallusto et al., Nature 401(6754): 708, 1999).

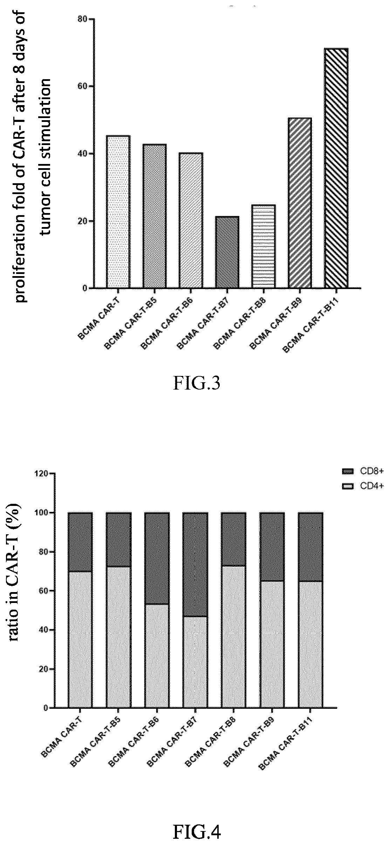

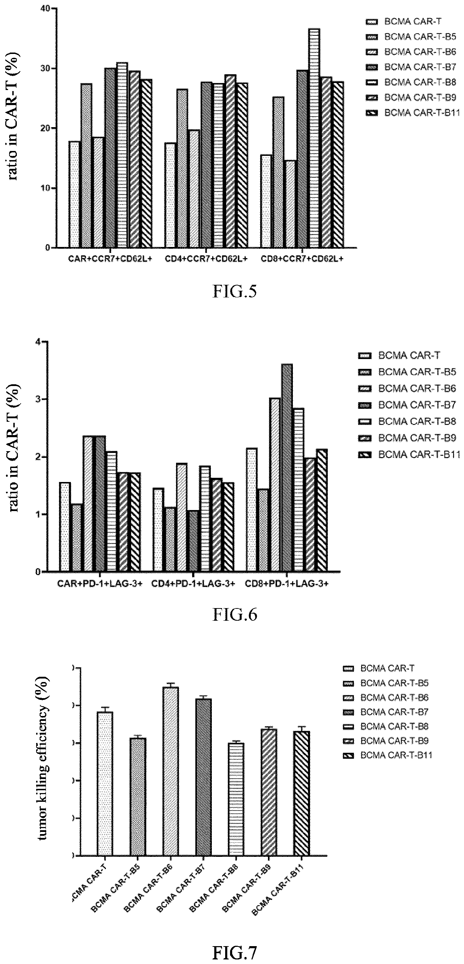

The results are shown in Figures 3 to 7. Figure 3 shows that BCMA CAR-T-B11 with the modified 4-1BB co-stimulatory signaling domain can accelerate the proliferation of CAR-T cells by about two-thirds.As a result, the killing speed of tumors can be increased in vivo, the time required for clinical cell preparation can be shortened in vitro, the waiting time of patients can be reduced, the chance of curing the disease can be increased, and the treatment cost of patients can be reduced. Figure 4 shows that BCMA CAR-T-B6 and BCMA CAR-T-B7 with the modified 4-1BB co-stimulatory signaling domain can approximately double the proportion of CD8⁺CAR-T cells in CAR-T cells, and thereby enhance the killing efficacy of the corresponding CAR-T cells on tumor cells. Figure 5 shows that BCMA CAR-T-B5, BCMA CAR-T-B7, BCMA CAR-T-B8, BCMA CAR-T-B9, and BCMA CAR-T-B11 with the modified 4-1BB co-stimulatory signaling domain can increase the proportion of CAR-Tcns in CAR-T cells by about 30%, which can increase the survival period of corresponding CAR-T cells in vivo and reduce the risk of disease recurrence. Figure 6 shows that the modified 4-1BB co-stimulatory domain has no significant effect on the depletion state of the corresponding CAR-T cells. It is shown that the modified 4-1BB has no effect on the depletion state of the cells while improving the killing efficiency and the survival cycle in vivo of the corresponding CAR-T cells. Figure 7 shows that BCMA CAR-T-B6 and BCMA CAR-T-B7 with the modified 4-1BB co-stimulatory signaling domain can increase the killing effect by about 10%, which is significant for helping patients achieve complete remission and reduce disease recurrence.

In conclusion, it can be seen that, after the modification of 4-1BB co-stimulatory signaling domain, CAR-T has significantly improved in vitro cell proliferation, subpopulation distribution, cell depletion and tumor cell killing.

This description is sufficient to enable those skilled in the art to practice the invention. The examples are provided for illustrative purposes only, and are not intended to limit the scope of the invention in any way. Indeed, according to the above description, in addition to those shown and described herein, various modifications of the invention will become apparent to those skilled in the art and are within the scope of the appended claims.

## Claims

1. A modified co-stimulatory domain from a member of the tumor necrosis factor receptor superfamily, **characterized in that**, compared to a native sequence, the modified co-stimulatory domain comprises a replacement of one or more amino acid residues within one or more motifs that interact with a downstream signaling molecule; optionally, the modified co-stimulatory domain comprises a replacement of one or more amino acid residues at the N-terminus and/or C-terminus of one or more motifs that interact with a downstream signaling molecule;
optionally, wherein the member of the tumor necrosis factor receptor superfamily is selected from the group consisting of CD30, CD40, CD27, OX40, 4-1BB, ATAR, LTβR and LMP1; and/or the downstream signaling molecule is selected from the group consisting of TRAF1, TRAF2, TRAF3, TRAF5 and TRAF6, in particular, wherein the member of the tumor necrosis factor receptor superfamily is 4-1BB and the downstream signaling molecule is TRAF2;
optionally, wherein the motif in the co-stimulatory domain from the member of the tumor necrosis factor receptor superfamily that interacts with a downstream signaling molecule is selected from the group consisting of (P/S/A/T)X(Q/E)E and PXQXXD, wherein X is any naturally occurring amino acid, and the replacement results in a non-naturally occurring motif at the modified position in the modified member of the tumor necrosis factor receptor superfamily;
optionally, wherein two different motifs exchange positions in the modified co-stimulatory domain.

2. The modified co-stimulatory domain of claim 1, wherein the member of the tumor necrosis factor receptor superfamily is 4-1BB, and the modified 4-1BB co-stimulatory domain comprises PTEE or PEEE motif replaced from TTQE motif and/or PEQE, TEQE or TTQE motif replaced from PEEE motif.

3. The modified co-stimulatory domain of claim 2, wherein the modified 4-1BB co-stimulatory domain comprises FPEEE motif replaced from QTTQE motif and/or QTTQE motif replaced from FPEEE motif.

4. The modified co-stimulatory domain of claim 2, wherein the modified 4-1BB co-stimulatory domain comprises, consists essentially of, or consists of the amino acid sequence set forth in SEQ ID NO: 4, 6 or 8.

5. A modified co-stimulatory domain from a member of the tumor necrosis factor receptor superfamily, **characterized in that**, compared to a native sequence, the modified co-stimulatory domain adds or deletes one or more motifs that interact with a downstream signaling molecule; optionally, the modified co-stimulatory domain adds or deletes one or more amino acid residues at the N-terminus and/or C-terminus of the motif that interacts with a downstream signaling molecule;
optionally, wherein the member of the tumor necrosis factor receptor superfamily is selected from the group consisting of CD30, CD40, CD27, OX40, 4-1BB, ATAR, LTβR and LMP1; and/or the downstream signaling molecule is selected from the group consisting of TRAF1, TRAF2, TRAF3, TRAF5 and TRAF6, in particular, wherein the member of the tumor necrosis factor receptor superfamily is 4-1BB and the downstream signaling molecule is TRAF2;
optionally, wherein the motif in the co-stimulatory domain from the member of the tumor necrosis factor receptor superfamily that interacts with a downstream signaling molecule is selected from the group consisting of (P/S/A/T)X(Q/E)E and PXQXXD, wherein X is any naturally occurring amino acid.

6. The modified co-stimulatory domain of claim 5, wherein the member of the tumor necrosis factor receptor superfamily is 4-1BB, and the modified 4-1BB co-stimulatory domain comprises additional TTQE and/or PEEE motifs.

7. The modified co-stimulatory domain of claim 6, wherein the modified 4-1BB co-stimulatory domain comprises additional VQTTQEEDGCS and/or RFPEEEEGGCE sequences.

8. The modified co-stimulatory domain of claim 6, wherein the modified 4-1BB co-stimulatory domain comprises, consists essentially of, or consists of the amino acid sequence set forth in SEQ ID NO: 10 or 12.

9. A modified co-stimulatory domain from a member of the tumor necrosis factor receptor superfamily, **characterized in that**, compared to a native sequence, one or more multimerization-related sequences from a downstream signaling molecule are added to the modified co-stimulatory domain; optionally, the multimerization is trimerization;
optionally, wherein the member of the tumor necrosis factor receptor superfamily is selected from the group consisting of CD30, CD40, CD27, OX40, 4-1BB, ATAR, LTβR and LMP1; and/or the downstream signaling molecule is selected from the group consisting of TRAF1, TRAF2, TRAF3, TRAF5 and TRAF6, in particular, wherein the member of the tumor necrosis factor receptor superfamily is 4-1BB and the downstream signaling molecule is TRAF2.

10. The modified co-stimulatory domain of claim 9, wherein the member of the tumor necrosis factor receptor superfamily is 4-IBB, and the modified 4-1BB co-stimulatory domain comprises the DLAMADLEQKV trimerization sequence from TRAF2.

11. The modified co-stimulatory domain of claim 10, wherein the modified 4-1BB co-stimulatory domain comprises, consists essentially of, or consists of the amino acid sequence set forth in SEQ ID NO: 14.

12. A method of modifying a co-stimulatory domain from a member of the tumor necrosis factor receptor superfamily, which comprises: replacing one or more amino acid residues within one or more motifs in the co-stimulatory domain that interact with a downstream signaling molecule; and/or adding or deleting one or more motifs in the co-stimulatory domain that interact with a downstream signaling molecule; and/or adding to the co-stimulatory domain one or more multimerization-related sequences from a downstream signaling molecule.

13. A chimeric antigen receptor, which comprises the modified co-stimulatory domain of any one of claims 1 to 11 or the modified co-stimulatory domain obtained by the method of claim 12.

14. An animal cell, which comprises the chimeric antigen receptor of claim 13.

15. A method of optimizing a chimeric antigen receptor comprising a co-stimulatory domain from a member of the tumor necrosis factor receptor superfamily, which comprises: replacing one or more amino acid residues within one or more motifs in the co-stimulatory domain from the member of the tumor necrosis factor receptor superfamily that interact with a downstream signaling molecule; and/or adding or deleting one or more motifs in the co-stimulatory domain from the member of the tumor necrosis factor receptor superfamily that interact with a downstream signaling molecule; and/or adding one or more multimerization-related sequences from a downstream signaling molecule to the co-stimulatory domain from the member of the tumor necrosis factor receptor superfamily.
